# EUROPÄISCHE PATENTSCHRIFT

(11) **EP 4 072 470 B1**
(45) Veröffentlichungstag und Bekanntmachung des Hinweises auf die Patenterteilung: **07.10.2026**
(21) Anmeldenummer: 20830096.2
(22) Anmeldetag: 10.12.2020
(51) Int. Cl.: A61F 2/16

(54) **OPHTHALMOLOGISCHES IMPLANTAT**
OPHTHALMOLOGICAL IMPLANT
IMPLANT OPHTALMOLOGIQUE

(30) Priorität: 13.12.2019 DE 102019134301
(43) Veröffentlichungstag der Anmeldung: 19.10.2022
(73) Patentinhaber: Carl Zeiss Meditec AG, 07745 Jena (DE)
(72) Erfinder: SCHREIBER, Benjamin, 10551 Berlin (DE)
(74) Vertreter: Hofstetter, Schurack & Partner
(86) Internationale Anmeldenummer: PCT/EP2020/085616
(87) Internationale Veröffentlichungsnummer: WO 2021/116332

(56) Entgegenhaltungen:
- US-A1- 2012 191 185
- US-A1- 2015 342 725
- US-B1- 9 358 103

## Beschreibung

### Technisches Gebiet

Die Erfindung betrifft ein ophthalmologisches Implantat.

### Stand der Technik

Bei der Kataraktoperation wird die natürliche menschliche Linse durch eine künstliche intraokulare Linse (IOL) ersetzt. Die IOL wird im Äquator des menschlichen Kapselsacks platziert. Dabei wird die vordere Seite des Kapselsacks durch einen kreisförmigen Schnitt geöffnet. Die menschliche Linse wird durch das Loch, die Kapsulorhexis, entfernt. Diese Kreiswunde bleibt nach der Operation offen. In diesem geschädigten Gewebe werden Zellen zur Wundheilung gebildet und wandern entlang der Innenseite des Kapselsacks. Hierdurch kann nach Kataraktoperationen in manchen Fällen eine sogenannte posteriore Kapselopazifikation (posterior capsule opacification, PCO, Cataracta secundaria) auftreten. Diese entsteht, da die verbleibenden Linsenepithelzellen (E-Zellen) in der äquatorialen Region des Kapselsacks mitotisch aktiv sind und zu Fibroblasten transformieren können. Diese lösen dann eine Art Wundheilung aus, wobei kollagenhaltiges Bindegewebe gebildet wird. Da manche Fibroplasten-Subtypen nicht nur an die Innenseite des Kapselsacks migrieren, sondern sich auch zusammenziehen können, kommt es zur Faltenbildung im Kapselsack. Die Trübung der Kapsel ist somit die Folge eines Wundheilungsprozesses und einer damit verbundenen Narbenbildung. Da die dadurch verursachte Linsentrübung andere Ursachen als die ursprüngliche Katarakt-Erkrankung besitzt, spricht man von einem "Nachstar" oder einem "sekundären Katarakt". Zusätzlich befinden sich Zellen auf der hinteren Seite des Kapselsacks im optischen Pfad und können Licht blockieren bzw. streuen, was ebenfalls die Sehschärfe reduziert. Ein klinisch signifikanter Nachstar kann bei den Betroffenen zu einer Minderung der Sehschärfe, der Farbwahrnehmung und des Kontrastsehens sowie zu einer erhöhten Blendung führen. Ohne die Implantation einer IOL in den leeren Kapselsack ist das Risiko eines Nachstars sogar noch erheblich erhöht, da in diesem Fall eine ungehinderte Zellmigration zur hinteren bzw. posterioren Oberfläche des Kapselsackes möglich ist.

Die Inzidenz eines sekundären Katarakts erhöht sich nach einem operativen Eingriff mit der Zeit. Eine Meta-Analyse der Fälle von Cataracta secundaria für alle bestehenden Arten von IOLs zeigte postoperativ eine ungefähre durchschnittliche Zunahme von 12% nach einem Jahr und eine ungefähre durchschnittliche Zunahme von 30% nach fünf Jahren. Ein entscheidender Faktor scheint hierbei auch das Alter des betreffenden Patienten zu sein, so dass damit gerechnet werden muss, dass fast alle behandelten Kinder und Jugendlichen nach einer gewissen Zeit unter postoperativem sekundären Katarakt leiden könnten.

Die US 2012/191185 A1 offenbart eine ophthalmische Intraokularlinse zur Förderung der Heilung und zur Hemmung der Hinterkammertrübung. Hierzu sind eine Intraokularlinse und eine Zusammensetzung vorgesehen, die an die Intraokularlinse gebunden oder darauf aufgetragen ist. Die Zusammensetzung enthält einen oder mehrere Wachstumsfaktoren und einen oder mehrere Antimitose-Wirkstoffe zur Hemmung der Hinterkammertrübung.

Die US 2015/342725 A1 offenbart ein intraokulares Implantat, das auf der Außenfläche eine Vielzahl von Musteroberflächenelementen aufweist, die in einem Abstand voneinander angeordnet sind und einen gewundenen Pfad definieren, dazu geeignet, einen Flüssigkeitsstrom oder einen Strom von in einer Flüssigkeit suspendierten Partikeln zu steuern, oder das Wachstum oder die Migration von Zellen zu hemmen.

Die US 9,358,103 B1 offenbart eine prothetische Kapselvorrichtung, die so konfiguriert ist, dass sie in ein Auge eingesetzt werden kann. Die Kapselvorrichtung umfasst eine Gehäusestruktur und eine Ringstruktur. Es existiert ein Hohlraum, der so gestaltet ist, dass er eine intraokulare Vorrichtung (z. B. eine IOL) enthält.

Es ist bisher nicht möglich, im Rahmen von Augenoperationen alle Epithelzellen quantitativ zu entfernen, ohne toxische Arzneimittel wie beispielsweise 5-Fluoruracil, Thapsigargin oder Paclitaxel zu verwenden, die die Endothelzellschicht der Hornhaut beschädigen oder anderes Augengewebe schädigen können. Die durch Fibrose und PCO hervorgerufenen Sehprobleme werden teilweise durch eine sogenannte fokale Nd:YAG-Kapsulotomie behandelt. Trotzdem können insbesondere fibrotische Striae weiterhin die Akkomodationsfähigkeit der Augenlinse erheblich beeinträchtigen.

### Darstellung der Erfindung

Aufgabe der vorliegenden Erfindung ist es, ein ophthalmologisches Implantat zu schaffen, welches das PCO- und Fibroserisiko verringert.

Die Aufgabe wird erfindungsgemäß durch ein ophthalmologisches Implantat gemäß Patentanspruch 1 gelöst. Vorteilhafte Ausgestaltungen mit zweckmäßigen Ausbildungen der Erfindung sind in den Unteransprüchen angegeben.

Ein erster Aspekt der Erfindung betrifft ein ophthalmologisches Implantat gemäß Anspruch 1, bei welchem erfindungsgemäß vorgesehen ist, dass dieses einen Grundkörper und wenigstens einen am Grundkörper immobilisierten Wachstumsfaktor aufweist, wobei der Wachstumsfaktor zum Stimulieren wenigstens eines Aspekts aus einer Gruppe, die Proliferation, Migration und Differenzierung von im menschlichen oder tierischen Auge vorkommenden Zellen umfasst, ausgebildet ist. Mit anderen Worten ist es im Gegensatz zu bekannten ophthalmologischen Implantaten vorgesehen, dass das Implantat das Zellwachstum und die Gewebebildung beschleunigt. Dies stellt eine vollständige Abkehr von bisherigen

Strategien dar, gemäß welchen ein Zellwachstum und eine Gewebebildung im Zusammenhang mit Kataraktoperationen möglichst unterbunden werden soll, um das Entstehen der PCO zu verhindern. Demgegenüber bewirkt das ophthalmologische Implantat der vorliegenden Erfindung nach seiner Implantation ein verstärktes Gewebewachstum durch eine aktive Zellkultivierung und fördert dadurch eine natürliche Wundheilung. Die Proliferation und Migration der im menschlichen oder tierischen Auge vorkommenden Zellen können damit kontrolliert werden, so dass diese Zellen die vorstehend genannten Probleme, die zur PCO führen, nicht länger verursachen können. Darüber hinaus ist die Induzierung und gegebenenfalls Steuerung einer schnellen Fibrose von Vorteil, da sie die Stabilität des Implantats erhöht, die endgültige Positionierung des Implantats im Kapselsack schnell sicherstellt und die Heilungsdauer nach der Kataraktoperation verkürzt. Vorzugsweise ist der wenigstens eine Wachstumsfaktor derart am Implantat immobilisiert, dass er das Wachstum bzw. die Migration von Zellen auf die Unterseite des Implantats bzw. die posteriore Seite des Kapselsacks erschwert, verlangsamt oder vorzugsweise vollständig verhindert. Die Immobilisierung des Wachstumsfaktors kann beispielsweise durch kovalente Anbindung, gegebenenfalls unter Verwendung eines Spacers, an das Implantatmaterial erfolgen. Dies kann in manchen Ausführungsformen beispielsweise durch Pfropfpolymerisation erreicht werden. Generell können aber auch andere Immobilisierungstechniken wie beispielweise interpenetrierende bzw. semi-/pseudo-interpenetrierende Netzwerke und dergleichen vorgesehen sein.

Weiterhin ist vorgesehen, dass das ophthalmologische Implantat einen Grundkörper mit wenigstens einem haptischen und wenigstens einem optischen Teil umfasst. Vorzugsweise ist der wenigstens eine Wachstumsfaktor dabei zumindest am haptischen Teil angeordnet. Hierdurch wird zuverlässig verhindert, dass die Funktionalität des optischen Teils durch den Wachstumsfaktor und etwaige weitere Verbindungen beeinträchtigt wird. Darüber hinaus besteht die Möglichkeit, den haptischen Teil als physikalische Barriere gegen Zellwanderung zu gestalten. Obwohl der Grundkörper in Abhängigkeit von der Ausgestaltung des ophthalmologischen Implantats grundsätzlich auch einteilig sein kann, bietet die Ausgestaltung mit einem haptischen und einem optischen Teil den weiteren Vorteil, dass der Grundkörper aus unterschiedlichen Materialien hergestellt sein kann, um die individuellen Anforderungen von Optik und Haptik besser berücksichtigen zu können. Vorzugsweise ist der Wachstumsfaktor derart angeordnet, dass die Zellen auf dem haptischen Teil "gefangen" werden bzw. sich nur entlang des haptischen Teils ausbreiten und insbesondere nicht den äquatorialen Bereich des Kapselsacks überqueren können.

Weiterhin ist vorgesehen, dass der Grundkörper zumindest bereichsweise feinstrukturiert ist. Eine Feinstrukturierung, bei der es sich beispielsweise um eine Mikro- und/oder Nanostrukturierung handeln kann, wird vorteilhaft zum gezielten Anhaften von Zellen sowie zur Förderung des Zellwachstums verwendet. Die feinstrukturierte Oberfläche umfasst konkave Bereichen und konvexe Bereichen in Form eines Rillenmusters, wobei das Rillenmuster ein periodisches Strukturelement bildet, wobei wenigstens ein Erstreckungsvektor der Periodizität des Strukturelements senkrecht zu einem Rand des optischen Teils angeordnet ist und wobei der Wachstumsfaktor am haptischen Teil an einer einem Rand des Grundkörpers zugewandten Seite der feinstrukturierten Oberfläche angeordnet ist. Mit anderen Worten ist es vorgesehen, dass die Feinstrukturierung entlang einer vorbestimmten Strecke eine mehrfach wiederkehrende Geometrie in Form eines Rillenmusters aufweist, die eine Regelmäßigkeit aufweist.

In einer weiteren vorteilhaften Ausgestaltung der Erfindung ist vorgesehen, dass das ophthalmologische Implantat wenigstens einen am Grundkörper angeordneten Haftvermittler, insbesondere Fibronectin, Vitronectin, Laminin und/oder ein Glycoprotein, zum Immobilisieren von Zellen am Grundkörper umfasst. Hierdurch kann das Zellwachstum ebenfalls gefördert, beschleunigt und gesteuert werden.

In einer weiteren vorteilhaften Ausgestaltung der Erfindung ist vorgesehen, dass der Grundkörper zumindest bereichsweise eine aufgeraute Oberfläche aufweist. Eine aufgeraute Oberfläche kann ebenfalls vorteilhaft zum gezielten Anhaften von Zellen sowie zur Förderung des Zellwachstums verwendet werden. Dabei sind beispielsweise mono- oder biaxial angeordnete oder zufällig angeordnete Oberflächenmodifikationen denkbar. Eine raue bzw. feinstrukturierte Oberfläche kann beispielsweise durch sogenannte Reverse-Epitaxie, Gießformen, Laser- oder Elektronenstrahlerosion, Nanoimprint Lithographie, Nanoembossing, molekulare und/oder partikuläre Selbstassemblierung und dergleichen sowie beliebige Kombinationen hieraus gezielt hergestellt werden. Alternativ oder zusätzlich ist es vorgesehen, dass der

Grundkörper zumindest bereichsweise scharfkantig ausgebildet ist. Unter einer scharfen Kante wird dabei eine Kante mit einem Winkel von höchstens 100°, insbesondere von höchstens 90° oder weniger verstanden. Hierdurch kann eine linienartige Anlage der Kante an angrenzenden biologischen Strukturen wie beispielsweise einer Wand des Kapselsacks erreicht werden, wodurch eine hohe Barrierewirkung gegen Zellwanderung erzielt wird.

Weitere Vorteile ergeben sich dadurch, dass die feinstrukturierte Oberfläche konkave Bereiche und konvexe Bereiche, die streifenförmig, zylinderförmig, kegelförmig, halbkugelförmig, quaderförmig, würfelförmig und/oder pyramidal ausgebildet und/oder in Form wenigstens eines Musters aus der Gruppe Zickzackmuster, Haifischmuster und Säulenmuster angeordnet sind, umfasst. Hierdurch können über eine geeignete Wahl der Geometrie der Feinstrukturierung Zellen zur

Steuerung des Zellwachstums gezielt in bestimmte Regionen des Implantats geleitet werden, um die Zellen dort gezielt anzusiedeln und zu vermehren. Umgekehrt kann durch entsprechende Ausgestaltung der Feinstrukturierung auch verhindert werden, dass Zellen an bestimmten Regionen des Implantats anhaften können. Beispielsweise kann die Feinstrukturierung als eine Art Barriere ausgebildet sein, die verhindert, dass Zellen sich beispielsweise am optischen Teil des Implantats anlagern können und zu Lichtstreuungen oder anderen optischen Störungen führen. Ebenso kann die Feinstrukturierung derart ausgebildet sein, dass eine Wanderung von Zellen über den äquatorialen Bereich des Implantats verhindert wird.

Zusätzlich ist vorgesehen, dass die feinstrukturierte Oberfläche zumindest bereichsweise auf dem haptischen Teil und konzentrisch zu einem Rand des optischen Teils ausgebildet ist. Beide Ausführungsformen stellen vorteilhafte Möglichkeiten zur Steuerung der Zellanhaftung und des Zellwachstums in bestimmten Bereichen des Implantats dar. Vorzugsweise ist das periodische Strukturelement bzw. die konzentrische Anordnung derart ausgestaltet, dass ein Zellwachstum radial zum optischen Teil verlangsamt und vorzugsweise eine Zellmigration über einen äquatorialen Rand des haptischen Teils verhindert wird.

Weitere Vorteile ergeben sich, indem ein Haftvermittler auf der feinstrukturierten Oberfläche und/oder an einer einem Rand des Grundkörpers zugewandten Seite der feinstrukturierten Oberfläche angeordnet ist. Mit anderen Worten wird wenigstens ein Haftvermittler mit der aufgerauten bzw. feinstrukturierten Oberfläche kombiniert und gezielt an bestimmten Oberflächenbereichen angeordnet bzw. immobilisiert, um die Zellanhaftung und das Zellwachstum an diesen Stellen zu befördern und zu steuern. Dies stellt eine weitere vorteilhafte Möglichkeit zur Kontrolle der durch das Implantat induzierten Wundheilung dar. Insbesondere kann hierdurch eine Zellmigration über den äquatorialen

Bereich des Implantats hinweg auf eine posteriore Seite des Kapselsacks verhindert werden.

In einer weiteren vorteilhaften Ausgestaltung der Erfindung ist vorgesehen, dass der Wachstumsfaktor in einem Randbereich des Grundkörpers angeordnet ist und/oder dass der Wachstumsfaktor zum Stimulieren wenigstens eines Aspekts aus der Gruppe Proliferation, Migration und Differenzierung von im Kapselsack eines menschlichen oder tierischen Auge vorkommenden Zellen, insbesondere von Linsenepithelzellen, ausgebildet ist. Auch hierdurch kann eine Zellwanderung über den Randbereich und damit eine Unterwanderung des Implantats zuverlässig verhindert werden. Der Wachstumsfaktor kann beispielsweise TGF-β (TGF-β1, TGF-β2, TGF-β3) sein oder umfassen. Die Zellen können dabei grundsätzlich auch in verschiedenen Zellformen, beispielsweise als Fibroplasten, und/oder als Zellansammlungen, beispielsweise als Wedl-Zellen auftreten.

In einer weiteren vorteilhaften Ausgestaltung der Erfindung ist vorgesehen, dass der Grundkörper zumindest teilweise aus wenigstens einem hydrophoben und/oder einem hydrophilen Polymer besteht und/oder zumindest bereichsweise eine hydrophobe oder eine hydrophile Oberfläche besitzt. Durch hydrophile Polymere bzw. Oberflächen können die Biokompatibilität des Implantats vorteilhaft gesteigert und die induzierte Wundheilung zusätzlich beschleunigt werden. Die Verwendung von hydrophoben Polymeren bzw. Oberflächen erschwert vorteilhaft eine Anlagerung von Zellen am Implantat.

Weitere Vorteile ergeben sich, indem das ophthalmologische Implantat als Intraokularlinse ausgebildet ist. Hierdurch können die vorstehend genannten Vorteile aufgrund der kontrollierten Zellmigration und Gewebeneubildung für unterschiedliche Implantattypen realisiert werden. Insbesondere kann eine Unterwanderung des Implantats durch Migration von Zellen über den äquatorialen Bereich des Implantats unterbunden werden.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Dabei zeigt:
- Fig. 1: eine schematische seitliche Schnittansicht eines menschlichen Auges, in dessen Kapselsack ein ophthalmologisches Implantat gemäß einem Ausführungsbeispiel der Erfindung angeordnet ist;
- Fig. 2: schematische Schnittansichten eines nicht-erfindungsgemäßen ophthalmologischen Implantats, das in den Kapselsack eines Auges implantiert ist und während der Wundheilung von Zellen unterwandert wird;
- Fig. 3: eine schematische Aufsicht eines erfindungsgemäßen ophthalmologischen Implantats mit einer Detailvergrößerung eines feinstrukturierten Oberflächenbereichs;
- Fig. 4: eine schematische seitliche Schnittansicht der in Fig. 3 gezeigten feinstrukturierten Oberfläche;
- Fig. 5: eine schematische Aufsicht der feinstrukturierten Oberfläche; und
- Fig. 6: schematische Schnittansichten des erfindungsgemäßen ophthalmologischen Implantats, das in den Kapselsack eines Auges implantiert ist und während der Wundheilung nicht von Zellen unterwandert wird.

### Bevorzugte Ausführung der Erfindung

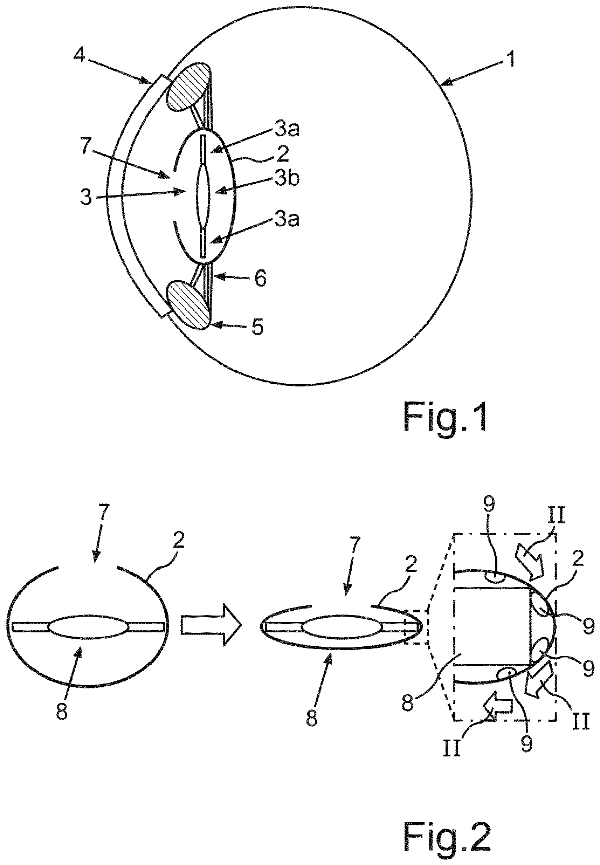

Fig. 1 zeigt eine schematische seitliche Schnittansicht eines menschlichen Auges 1, in dessen Kapselsack 2 im Rahmen einer Kataraktoperation ein ophthalmologisches Implantat 3 gemäß einem Ausführungsbeispiel der Erfindung angeordnet ist. Vom Auge 1 sind in schematischer Weise auch die Hornhaut 4, die Ziliarkörper 5 und die Zonulafasern 6 näher gezeigt. Bei der Kataraktoperation wird zunächst die natürliche menschliche Linse durch das Implantat 3 ersetzt, das vorliegend als Intraokularlinse (IOL) mit einem haptischen Teil 3a und einem optischen Teil 3b ausgebildet ist. Die IOL 3 wird im Äquator des Kapselsacks 2 platziert. Dabei wird die vordere Seite des Kapselsacks 2 durch einen kreisförmigen Schnitt geöffnet, wie dies in Fig. 1 gezeigt ist. Die menschliche Linse wird durch das gebildete Loch, die Kapsulorhexis 7, entfernt. Diese Kreiswunde bleibt nach der Operation offen. In diesem geschädigten Gewebe werden Zellen 9 (s. Fig. 2) zur Wundheilung gebildet und wandern entlang der Innenseite des Kapselsacks 2. Hierdurch kann nach Kataraktoperationen in manchen Fällen eine sogenannte posteriore Kapselopazifikation (posterior capsule opacification, PCO, Cataracta secundaria) auftreten. Zur Entstehung der PCO existiert die sogenannte Sandwichtheorie. Die Sandwichtheorie zur PCO-Bildung basiert auf den folgenden Annahmen:
1) Das epitheliale Einwachsen kann durch eine bioaktive Verbindung zwischen Implantat und Hornhautzellen behindert werden.
2) Ein Implantat kann biokompatibel sein (auf bioinerte oder bioaktive Weise).
3) Eine klinisch klare hintere Kapsel kann eine Monoschicht von Epithelzellen aufweisen.

Gemäß der Sandwichtheorie würde es ein Implantat aus einem bioaktivem Material einer einzelnen Linsenepithelzelle erlauben, sich sowohl an das Implantat als auch an die hintere Kapselwand zu binden. Dies würde ein Sandwichmuster mit dem Implantat, der Zellmonoschicht und der hinteren Kapsel ergeben. Diese Art der bioaktiven Bindung behindert dann das weitere Zellwachstum, da jede Zelle auf beiden Seiten eine biologische Bindung aufweist. Die derart versiegelte Sandwichstruktur sollte dann ein weiteres Einwachsen bzw. Wandern von Epithelzellen verhindern. Der Grad der Bioaktivität des Implantats könnte daher auch den grundlegenden Unterschied in den PCO-Häufigkeiten unterschiedlicher IOL-Materialien erklären.

Fig. 2 zeigt schematische Schnittansichten eines aus nicht-erfindungsgemäßen ophthalmologischen Implantats 8, das in den Kapselsack 2 eines Auges 1 implantiert ist und im Rahmen einer unkontrollierten Fibrose innerhalb von weniger als sechs Monaten von Zellen 9 unterwandert wird, da es nicht mit einem Wachstumsfaktor 11 ausgestattet ist. Man erkennt, dass die Zellen 9 ausgehend von der Kapsulorhexis 7 entlang der Wand des Kapselsacks 2 wandern und dabei gemäß der mit Pfeilen II gekennzeicheten Wanderungsrichtung den äquatorialen Rand des Implantats 8 bzw. den Äquator des Kapselsacks 2 überschreiten und in den posterioren Raum des Kapselsacks 2 vordringen, wobei sie das Implantat 8 unterwandern und eine PCO verursachen.

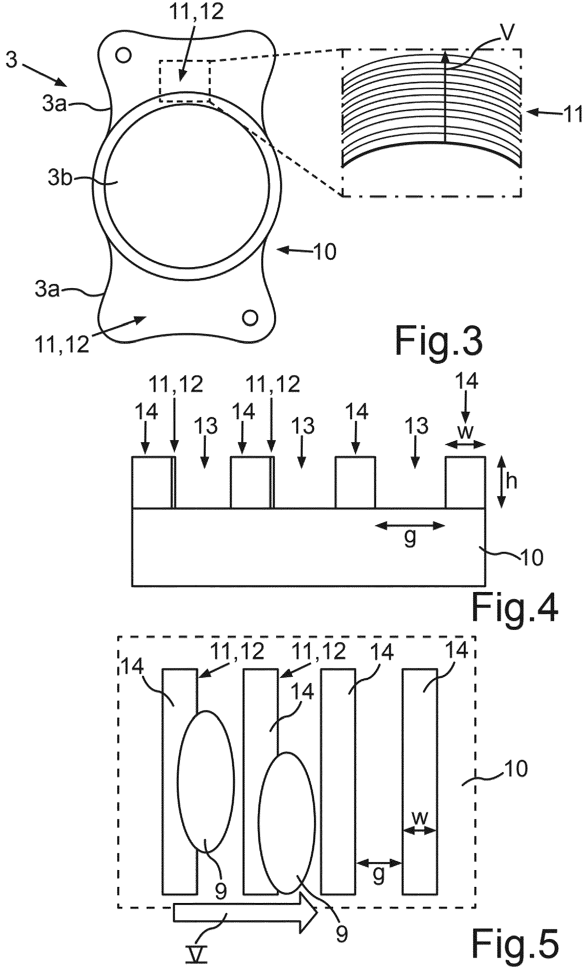

Fig. 3 zeigt eine schematische Aufsicht eines erfindungsgemäßen ophthalmologischen Implantats 3 mit einer Detailvergrößerung eines feinstrukturierten Oberflächenbereichs. Man erkennt, dass das Implantat 3 einen Grundkörper 10 mit einem zentralen, runden optischen Teil 3b und zwei haptischen Teilen 3a umfasst, wobei die haptischen Teile 3a an ihren am Kapselsack 2 anliegenden Enden jeweils scharfkantig (etwa 90°-Winkel) ausgebildet sind und dadurch fest am Kapselsack 2 anliegen und eine zusätzliche mechanische Barriere gegen Zellwanderung bilden. Zur Verhinderung von PCO ist das Implantat 3 dazu ausgebildet, ein kontrolliertes Zellwachstum sowie eine kontrollierte Zellmigration und Gewebeneubildung zu bewirken, um nach der Implantation eine kontrollierte und beschleunigte Fibrose sicherzustellen. Hierzu weist das Implantat 3 einen am Grundkörper 10 auf den haptischen Teilen 3a immobilisierten Wachstumsfaktor 11 zum Stimulieren wenigstens eines Aspekts aus einer Gruppe, die Proliferation, Migration und Differenzierung von im menschlichen oder tierischen Auge 1 vorkommenden Zellen 9 umfasst, auf. Bei dem Wachstumsfaktor 11 handelt es sich beispielsweise um TGF-β, wobei grundsätzlich auch andere geeignete Wachstumsfaktoren vorgesehen sein können. Zusätzlich kann vorgesehen sein, dass das Implantat 3 wenigstens einen am Grundkörper 10 angeordneten Haftvermittler 12 (s. Fig. 4), beispielsweise Fibronectin, Vitronectin, Laminin und/oder ein Glycoprotein, zum Immobilisieren von Zellen 9 am Grundkörper 10 umfasst. Hierdurch kann eine zusätzliche Steuerung des Zellwachstums und der Zellmigration erreicht werden.

Wie man an der Detailvergrößerung und in Fig. 4 und Fig. 5 sieht, ist der Grundkörper 10 zudem bereichsweise feinstrukturiert. Im gezeigten Ausführungsbeispiel handelt es sich dabei um Nanostrukturen mit konkaven Bereichen 13 und quaderförmigen konvexen Bereichen 14, die gemeinsam ein Rillenmuster bilden. Die Breite der konvexen Bereiche 14 ist mit w, die Höhe mit h und der Abstand benachbarter konvexer Bereiche 14 mit g bezeichnet. Die genannten Bemaßungen w, h, g sind im gezeigten Beispiel jeweils identisch für die einzelnen Bereiche 13, 14 gewählt und betragen jeweils 5 µm, wodurch das Rillenmuster ein periodisches Strukturelement bildet, dessen Erstreckungsvektor v senkrecht zu einem Rand des optischen Teils 3b angeordnet ist. Zudem ist das Rillenmuster konzentrisch zu einem Rand des optischen Teils 3b ausgebildet. Generell können die Bemaßungen w, h und/oder g sowie die Form und Anzahl der konkaven und konvexen Bereiche 13, 14 unterschiedlich bzw. variierend gewählt werden. Ebenso kann vorgesehen sein, dass abweichende Geometrien für die konkaven und/oder konvexen Bereiche 13, 14 gewählt werden. Durch diese Mikro- bzw. Nanostrukturierung wird eine Zellmigration in Richtung der Kanten des Implantats 3 bzw. dem Äquator des Kapselsacks 2 stark reduziert bzw. im Idealfall sogar vollständig unterbunden. Allerdings ist festzustellen, dass eine mikro- oder nanostrukturierte Oberfläche in Abhängigkeit des Implantatmaterials nur in Verbindung mit dem wenigstens einen Wachstumsfaktor 11 die Barrierewirkung verbessern kann, da Zellen 9 von sich aus nicht an allen Materialien haften. So sind beispielsweise hydrophile Materialien, wie sie zur Herstellung von IOLs verwendet werden, im allgemeinen nicht haftend, so dass eine Nanostrukturierung ohne Wachstumsfaktor 11 nicht funktionieren würde. Häufig genügt auch nur eine erhöhte Oberflächenrauheit bzw. eine ungeordnete Oberflächenstruktur, vorzugsweise auf den haptischen Teilen 3a, um in Verbindung mit dem Wachstumsfaktor 11 eine Verlangsamung oder Unterbindung der Zellmigration in Richtung Äquator des Kapselsacks 2 zu bewirken.

Fig. 4 zeigt eine schematische seitliche Schnittansicht der in Fig. 3 gezeigten feinstrukturierten Oberfläche, während Fig. 5 eine schematische Aufsicht der feinstrukturierten Oberfläche zeigt. Man erkennt, dass der Wachstumsfaktor 11 und der grundsätzlich optionale Haftvermittler 12 nur an in Richtung eines Rands der haptischen Teile 3a zeigenden Flanken derjenigen konvexen Bereiche 14 immobilisiert sind, die räumlich in der Nähe des optischen Teils 3b liegen. Hierdurch werden Zellen 9, die ausgehend von der Kapsulorhexis 7 bzw. vom optischen Teil 3b in Richtung der Ränder der haptischen Teile 3a und damit in Richtung des Äquators des Implantats 3 bzw. des Kapselsacks 2 wandern (vgl. Fig. 5, Pfeil V), bereits in der Nähe des optischen Teils 3b gestoppt und zur Proliferation, Migration und/oder Differenzierung angeregt. Hierdurch wird in unmittelbarer Nähe des optischen Teils 3b eine natürliche "Zellbarriere" gemäß der Sandwichtheorie erzeugt, die zu einer erheblichen Verringerung des PCO-Risikos führt, ohne dass hierfür stark toxische Medikamente benötigt würden. Anstelle von gegebenenfalls periodischen Nanostrukturen genügt häufig auch eine erhöhte Oberflächenrauheit in Kombination mit wenigstens einem Wachstumsfaktor 11 und gegebenenfalls einem zusätzlichen Haftvermittler 12 bzw. Zelladhäsionsförderer.

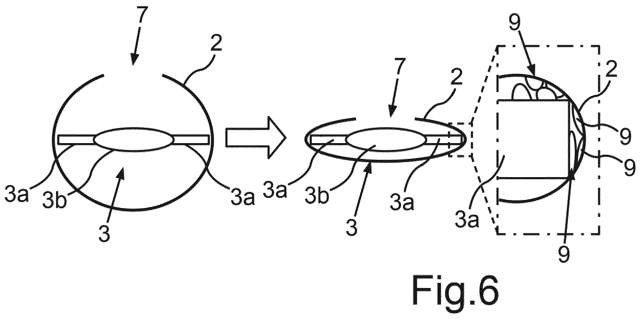

Fig. 6 zeigt schematische Schnittansichten des erfindungsgemäßen ophthalmologischen Implantats 3, das in den Kapselsack 2 eines Auges 1 implantiert ist und aufgrund der vorstehend beschriebenen Eigenschaften im Unterschied zum in Fig. 2 gezeigten Implantat 8 während der Wundheilung nicht von Zellen 9 unterwandert wird. Stattdessen bilden die Zellen 9 natürliche "Zellbarrieren" und fixieren das Implantat 3 am Kapselsack 2. Durch das erfindungsgemäße Konzept des gezielt induzierten und verstärkten Gewebewachstums wird die Situation der natürlichen Wundheilung imitiert und beschleunigt und so die Proliferation und Migration der Zellen 9 gestoppt. Darüber hinaus ist eine kontrollierte und schnellere Fibrose von Vorteil, da sie die Stabilität des Implantats 3 erhöht und die Heilungszeiträume nach der Operation verkürzt.

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, Systemfehlern, DIN-Toleranzen und dergleichen - als vom Rahmen der Erfindung mitumfasst anzusehen.

### Bezugszeichenliste

- 1: Auge
- 2: Kapselsack
- 3: opthalmologisches Implantat
- 3a: haptischer Teil
- 3b: optischer Teil
- 4: Hornhaut
- 5: Ziliarkörper
- 6: Zonulafasern
- 7: Kapsulorhexis
- 8: nicht-erfindungsgemäßes Implantat
- 9: Zellen
- 10: Grundkörper
- 11: Wachstumsfaktor
- 12: Haftvermittler
- 13: konkaver Bereich
- 14: konvexer Bereich
- v: Vektor
- II, V: Wanderungsrichtung

## Patentansprüche

1. Ophthalmologisches Implantat (3) mit einem Grundkörper (10) mit wenigstens einem haptischen Teil (3a) und wenigstens einem optischen Teil (3b) und mit wenigstens einem am Grundkörper (10) immobilisierten Wachstumsfaktor (11) zum Stimulieren wenigstens eines Aspekts aus einer Gruppe, die:
- Proliferation von im menschlichen oder tierischen Auge vorkommenden Zellen (9),
- Migration von im menschlichen oder tierischen Auge vorkommenden Zellen (9) und
- Differenzierung von im menschlichen oder tierischen Auge vorkommenden Zellen (9)
umfasst,
wobei der Grundkörper (10) zumindest bereichsweise eine feinstrukturierte Oberfläche mit konkaven Bereichen (13) und konvexen Bereichen (14) umfasst, **dadurch gekennzeichnet, dass**
der Grundkörper (10) zumindest bereichsweise eine feinstrukturierte Oberfläche mit konkaven Bereichen (13) und konvexen Bereichen (14) in Form eines Rillenmusters umfasst, wobei das Rillenmuster ein periodisches Strukturelement bildet, wobei wenigstens ein Erstreckungsvektor (v) der Periodizität des Strukturelements senkrecht zu einem Rand des optischen Teils (3b) angeordnet ist und wobei der Wachstumsfaktor (11) am haptischen Teil an einer einem Rand des Grundkörpers (10) zugewandten Seite der feinstrukturierten Oberfläche angeordnet ist.

2. Ophthalmologisches Implantat (3) nach Anspruch 1,
**dadurch gekennzeichnet, dass**
dieses wenigstens einen am Grundkörper (10) angeordneten Haftvermittler (12) zum Immobilisieren von Zellen (9) am Grundkörper (10) umfasst.

3. Ophthalmologisches Implantat (3) nach Anspruch 2,
**dadurch gekennzeichnet, dass**
der Haftvermittler (12) Fibronectin, Vitronectin, Laminin und/oder ein Glycoprotein ist.

4. Ophthalmologisches Implantat (3) nach einem der Ansprüche 1 bis 3,
**dadurch gekennzeichnet, dass**
der Grundkörper (10) zumindest bereichsweise eine aufgeraute Oberfläche aufweist und/oder zumindest bereichsweise scharfkantig ausgebildet ist.

5. Ophthalmologisches Implantat (3) nach Anspruch 4,
**dadurch gekennzeichnet, dass**
die feinstrukturierte Oberfläche konkave Bereiche (13) und konvexe Bereiche (14), die streifenförmig, zylinderförmig, kegelförmig, halbkugelförmig, quaderförmig, würfelförmig und/oder pyramidal ausgebildet und/oder in Form wenigstens eines Musters aus der Gruppe Zickzackmuster, Haifischmuster und Säulenmuster angeordnet sind, umfasst.

6. Ophthalmologisches Implantat (3) nach einem der Ansprüche 1 bis 5,
**dadurch gekennzeichnet, dass**
die feinstrukturierte Oberfläche zumindest bereichsweise auf dem haptischen Teil (3a) und konzentrisch zu einem Rand des optischen Teils (3b) ausgebildet ist.

7. Ophthalmologisches Implantat (3) nach einem der Ansprüche 1 bis 6,
**dadurch gekennzeichnet, dass**
ein Haftvermittler (12) auf der feinstrukturierten Oberfläche und/oder an einer einem Rand des Grundkörpers (10) zugewandten Seite der feinstrukturierten Oberfläche angeordnet ist.

8. Ophthalmologisches Implantat (3) nach einem der Ansprüche 1 bis 7,
**dadurch gekennzeichnet, dass**
der Wachstumsfaktor (11) in einem Randbereich des Grundkörpers (10) angeordnet ist und/oder dass der Wachstumsfaktor (11) zum Stimulieren wenigstens eines Aspekts aus der Gruppe:
- Proliferation von im Kapselsack (2) eines menschlichen oder tierischen Auge (1) vorkommenden Zellen, insbesondere von Linsenepithelzellen,
- Migration von im Kapselsack (2) eines menschlichen oder tierischen Auge (1) vorkommenden Zellen, insbesondere von Linsenepithelzellen, und
- Differenzierung von im Kapselsack (2) eines menschlichen oder tierischen Auge (1) vorkommenden Zellen, insbesondere von Linsenepithelzellen,
ausgebildet ist.

9. Ophthalmologisches Implantat (3) nach einem der Ansprüche 1 bis 8,
**dadurch gekennzeichnet, dass**
der Grundkörper (10) zumindest teilweise aus wenigstens einem hydrophilen und/oder hydrophoben Polymer besteht und/oder zumindest bereichsweise eine hydrophile oder hydrophobe Oberfläche besitzt.

10. Ophthalmologisches Implantat (3) nach einem der Ansprüche 1 bis 9,
**dadurch gekennzeichnet, dass**
dieses als Intraokularlinse ausgebildet ist.

## Claims

1. Ophthalmological implant (3) having a main body (10) having at least one haptic part (3a) and at least one optical part (3b) and having at least one growth factor (11) immobilized on the main body (10) for stimulating at least one aspect from a group comprising:
- proliferation of cells (9) present in the human or animal eye,
- migration of cells (9) present in the human or animal eye, and
- differentiation of cells (9) present in the human or animal eye,
wherein the main body (10) comprises, at least in regions, a finely structured surface with concave regions (13) and convex regions (14), **characterized in that**
the main body (10) comprises, at least in regions, a finely structured surface with concave regions (13) and convex regions (14) in the form of a groove pattern, wherein the groove pattern forms a periodic structure element, wherein at least one extension vector (v) of the periodicity of the structure element is arranged perpendicular to an edge of the optical part (3b), and wherein the growth factor (11) is arranged on the haptic part on a side of the finely structured surface facing an edge of the main body (10).

2. Ophthalmological implant (3) according to Claim 1,
**characterized in that**
it comprises at least one adhesion promoter (12) arranged on the main body (10) for immobilizing cells (9) on the main body (10).

3. Ophthalmological implant (3) according to Claim 2,
**characterized in that**
the adhesion promoter (12) is fibronectin, vitronectin, laminin and/or a glycoprotein.

4. Ophthalmological implant (3) according to any of Claims 1 to 3,
**characterized in that**
the main body (10) has a roughened surface at least in regions and/or has a sharp-edged form at least in regions.

5. Ophthalmological implant (3) according to Claim 4,
**characterized in that**
the finely structured surface comprises concave regions (13) and convex regions (14) which are strip-shaped, cylindrical, conical, hemispherical, parallelepipedal, cubic and/or pyramidal and/or are arranged in the form of at least one pattern from the group of zigzag pattern, shark pattern and column pattern.

6. Ophthalmological implant (3) according to any of Claims 1 to 5,
**characterized in that**
the finely structured surface is formed at least in regions on the haptic part (3a) and concentrically with respect to an edge of the optical part (3b).

7. Ophthalmological implant (3) according to any of Claims 1 to 6,
**characterized in that**
an adhesion promoter (12) is arranged on the finely structured surface and/or on a side of the finely structured surface facing an edge of the main body (10).

8. Ophthalmological implant (3) according to any of Claims 1 to 7,
**characterized in that**
the growth factor (11) is arranged in an edge region of the main body (10) and/or **in that** the growth factor (11) is designed for stimulating at least one aspect from the group of:
- proliferation of cells present in the capsular bag (2) of a human or animal eye (1), in particular of lens epithelial cells,
- migration of cells present in the capsular bag (2) of a human or animal eye (1), in particular of lens epithelial cells, and
- differentiation of cells present in the capsular bag (2) of a human or animal eye (1), in particular of lens epithelial cells.

9. Ophthalmological implant (3) according to any of Claims 1 to 8,
**characterized in that**
the main body (10) at least partly consists of at least one hydrophilic and/or hydrophobic polymer and/or has a hydrophilic or hydrophobic surface at least in regions.

10. Ophthalmological implant (3) according to any of Claims 1 to 9,
**characterized in that**
it is embodied as an intraocular lens.

## Revendications

1. Implant ophtalmologique (3) présentant un corps de base (10) pourvu d'au moins une partie haptique (3a) et d'au moins une partie optique (3b) et au moins un facteur de croissance (11) immobilisé sur le corps de base (10), pour la stimulation d'au moins un aspect d'un groupe comprenant :
- la prolifération de cellules (9) se trouvant dans l'œil humain ou animal,
- la migration de cellules (9) se trouvant dans l'œil humain ou animal et
- la différenciation de cellules (9) se trouvant dans l'œil humain ou animal,
le corps de base (10) comprenant au moins par zones une surface finement structurée pourvue de zones concaves (13) et de zones convexes (14), **caractérisé en ce que**
le corps de base (10) comprend au moins par zones une surface finement structurée pourvue de zones concaves (13) et de zones convexes (14) sous la forme d'un motif à rainures, le motif à rainures formant un élément structural périodique, au moins un vecteur d'extension (v) de la périodicité de l'élément structural étant agencé perpendiculairement à un bord de la partie optique (3b) et le facteur de croissance (11) étant agencé au niveau de la partie haptique sur un côté orienté vers un bord du corps de base (10) de la surface finement structurée.

2. Implant ophtalmologique (3) selon la revendication 1,
**caractérisé en ce que**
celui-ci comprend au moins un promoteur d'adhérence (12), destiné à immobiliser des cellules (9) sur le corps de base (10), agencé sur le corps de base (10).

3. Implant ophtalmologique (3) selon la revendication 2,
**caractérisé en ce que**
le promoteur d'adhérence (12) est la fibronectine, la vitronectine, la laminine et/ou une glycoprotéine.

4. Implant ophtalmologique (3) selon l'une des revendications 1 à 3,
**caractérisé en ce que**
le corps de base (10) présente par zones au moins une surface rendue rugueuse et/ou est réalisé au moins par zones avec des arêtes vives.

5. Implant ophtalmologique (3) selon la revendication 4,
**caractérisé en ce que**
la surface finement structurée comprend des zones concaves (13) et des zones convexes (14) qui sont réalisées sous forme de bandes, cylindrique, conique, hémisphérique, parallélépipédique, cubique et/ou pyramidale et/ou qui sont agencées sous forme d'au moins un motif du groupe de motif en zigzag, motif en requin et motif colonnaire.

6. Implant ophtalmologique (3) selon l'une des revendications 1 à 5,
**caractérisé en ce que**
la surface finement structurée est réalisée au moins par zones sur la partie haptique (3a) et de manière concentrique par rapport à un bord de la partie optique (3b).

7. Implant ophtalmologique (3) selon l'une des revendications 1 à 6,
**caractérisé en ce que**
un promoteur d'adhérence (12) est agencé sur la surface finement structurée et/ou sur un côté orienté vers un bord du corps de base (10) de la surface finement structurée.

8. Implant ophtalmologique (3) selon l'une des revendications 1 à 7,
**caractérisé en ce que**
le facteur de croissance (11) est agencé dans une zone de bord du corps de base (10) et/ou **en ce que** le facteur de croissance (11) est réalisé pour la stimulation d'au moins un aspect du groupe :
- prolifération de cellules se trouvant dans le sac capsulaire (2) d'un œil (1) humain ou animal, en particulier de cellules épithéliales de la lentille,
- migration de cellules se trouvant dans le sac capsulaire (2) d'un œil (1) humain ou animal, en particulier de cellules épithéliales de la lentille et
- différenciation de cellules se trouvant dans le sac capsulaire (2) d'un œil (1) humain ou animal, en particulier de cellules épithéliales de la lentille.

9. Implant ophtalmologique (3) selon l'une des revendications 1 à 8,
**caractérisé en ce que**
le corps de base (10) est constitué au moins partiellement d'au moins un polymère hydrophile et/ou hydrophobe et/ou présente au moins par zones une surface hydrophile ou hydrophobe.

10. Implant ophtalmologique (3) selon l'une des revendications 1 à 9,
**caractérisé en ce que**
celui-ci est réalisé sous forme de lentille intraoculaire.
